# EUROPÄISCHE PATENTSCHRIFT

(11) **EP 0 673 249 B1**
(45) Veröffentlichungstag und Bekanntmachung des Hinweises auf die Patenterteilung: **17.03.1999**
(21) Anmeldenummer: 94902687.6
(22) Anmeldetag: 06.12.1993
(51) Int. Cl.: A61K 31/505

(54) **TRIAZOLOCHINAZOLINE ZUR BEHANDLUNG ZENTRALNERVÖSER ERKRANKUNGEN**
TRIAZOLOQUINAZOLINES FOR THE TREATMENT OF DISEASES OF THE CENTRAL NERVOUS SYSTEM
TRIAZOLOCHINAZOLINES UTILISEES DANS LE TRAITEMENT DE MALADIES DU SYSTEME NERVEUX CENTRAL

(30) Priorität: 10.12.1992 DE 4241564
(43) Veröffentlichungstag der Anmeldung: 27.09.1995
(73) Patentinhaber: BASF Aktiengesellschaft, 67063 Ludwigshafen (DE)
(72) Erfinder: SCHLECKER, Rainer, D-67281 Bissersheim (DE); TREIBER, Hans-Jörg, D-68782 Brühl (DE); BEHL, Berthold, D-67063 Ludwigshafen (DE); HOFMANN, Hans Peter, D-67117 Limburgerhof (DE)
(86) Internationale Anmeldenummer: EP9303424
(87) Internationale Veröffentlichungsnummer: WO9413294

(56) Entgegenhaltungen:
- EP-A- 0 080 176
- EP-A- 0 181 282
- J. MED. CHEM. Bd. 34 , 1991 Seiten 281 - 290 J.E. FRANCIS ET AL. 'Synthesis and benzodiazepine binding activity of a series of novel [1,2,4]triazoloè1,5-c]quin azolin-5(6H)-ones'

## Beschreibung

Die vorliegende Erfindung betrifft die Verwendung von Triazolochinazolinen zur Behandlung von zentralnervösen Erkrankungen.

Triazolochinazoline sind bereits aus der EP-A-0080 176 bekannt. Für diese Verbindungen wird eine antiallergische Wirkung angegeben. Es ist weiter bekannt, daß bestimmte Pyrazolochinazoline sich unter anderem zur Behandlung von neurologischen Störungen eignen (US-A-5 153 196).

Gegenstand der Erfindung ist die Verwendung von Triazolochinazolinen der Formel I, 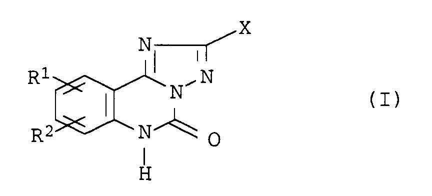 in der
- X: eine Carboxylgruppe gegebenenfalls in Form ihres Salzes mit einem physiologisch verträglichen Amin- oder Metallkation oder den Rest 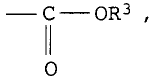 wobei R³ einen C₁₋₈-Alkylrest bedeutet,
- R¹: ein Wasserstoff-, Chlor- oder Bromatom, oder einen Trifluormethyl-, Nitro- oder C₁₋₅-Alkylrest und
- R²: ein Chlor- oder Bromatom oder einen Triflourmethyl-, Nitro- oder C₁₋₅-Alkylrest
bedeuten sowie deren physiologisch verträglichen Säureadditionssalzen zur Herstellung von Arzneimitteln zur Behandlung zentralnervöser Erkrankungen.

Die Verbindungen der Formel I sind in der EP-A-0080 176 beschrieben. Dort ist auch eine Vielzahl von Verbindungen aufgeführt, die sich auch für die neue Indikation eignen.

Als zentralnervöse Erkrankungen kommen beispielsweise Epilepsie, Hirnschädigungen, Morbus Parkinson, Morbus Alzheimer, Emesis, Kopf- und Rückenmarkstrauma in Betracht. Die Verbindungen der Formel I besitzen weiter den Vorteil, daß sie spasmolytische, antiepileptische, anxiolytische und antidepressive Eigenschaften besitzen. Die Wirkung der Verbindungen beruht auf deren glutamatantagonistischen Eigenschaften.

Die pharmakologische Wirksamkeit der erfindungsgemäß verwendeten Verbindungen I wurde an isoliertem Membranmaterial von Rattengroßhirnen untersucht. Hierzu wurde das Membranmaterial in Gegenwart der erfindungsgemäß verwendeten Verbindungen mit den radioaktiv markierten Substanzen ³H-2-Amino-3-hydroxy-5-methyl-4-isoxazolpropionsäure (³H-AMPA) und ³H-5,7-Dichlorkynurensäure behandelt, wobei sich diese an spezifische Rezeptoren (AMPA- bzw. NMDA-Rezeptor (N-Methyl-D-aspartat)) binden. Anschließend wurde durch Scintillationszählung die Radioaktivität der behandelten Membrane gemessen. Über die gebundene Radioaktivität ließen sich die Mengen an gebundener ³H-AMPA und ³H-5,7-Dichlorkynurensäure bzw. jeweils die verdrängten Mengen dieser radioaktiv markierten Substanzen bestimmen. Die sich hieraus ergebende Dissoziationskonstante K_{I} (I = Inhibitor), welche ein Maß für die Verdrängungswirkung des erfindungsgemäß verwendeten Wirkstoffes ist, wurde durch iterative nichtlineare Regressionsanalyse mit dem Statistical Analysis System (SAS) an einem IBM-Rechner, ähnlich dem Programm "Ligand" von P.J. Munson und D. Rodbard (Analytical Biochem. 107, 220 (1980), Ligand: Versatile Computerized Approach for Charakterization of Ligand Binding Systems) ermittelt.

Folgende In-Vitro-Untersuchungen wurden durchgeführt:

### 1. Bindung von ³H-2-Amino-3-hydroxy-5-methyl-4-isoxazolpropion-säure (³H-AMPA)

Für die Präparation des Membranmaterials wurden frisch entnommene Rattengroßhirne zusammen mit dem 15fachen Volumen einer Pufferlösung A aus 30 mM α,α,α-Tris-(hydroxymethyl)-methylamin-Hydrochlorid (TRIS-HCl) und 0,5 mM Ethylendiamintetraessigsäure (EDTA) - pH 7,4 - mittels eines Ultra-TURRAX-Rührers homogenisiert. Die Suspension wurde 20 Minuten bei 48 000 g zentrifugiert. Nach Abtrennung der überstehenden Flüssigkeit wurde das im Bodensatz enthaltene proteinhaltige Membranmaterial dreimal durch Suspendieren in der Pufferlösung A und anschließendes, jeweils 20 minütiges Zentrifugieren bei 48 000 g gewaschen. Danach wurde das Membranmaterial in einem 15fachen Volumen der Pufferlösung A suspendiert und 30 Minuten bei 37°C inkubiert. Anschließend wurde das Proteinmaterial zweimal durch Zentrifugieren und Suspendieren gewaschen und bis zur Verwendung bei -70°C eingefroren.

Für den Bindungstest wurde das bei 37°C aufgetaute Proteinmaterial zweimal durch Zentrifugieren bei 48 000 g (20 Minuten) und anschließendes Suspendieren in einer Pufferlösung B aus 50 mM TRIS-HCl, 0,1 M Kaliumthiocyanat und 2,5 mM Calciumchlorid - pH 7,1 - gewaschen. Anschließend wurden 0,25 mg Membranmaterial, 0,1 µCi ³H-AMPA (60 Ci/mmol) sowie Verbindung I in 1 ml Pufferlösung B gelöst und 60 Minuten auf Eis inkubiert. Die inkubierte Lösung wurde über einen CF/B-Filter (Firma Whatman), der zuvor mindestens 2 Stunden mit einer 0,5 %igen wäßrigen Lösung von Polyethylenimin behandelt worden war, filtriert. Anschließend wurde das Filtrat mit 5 ml kalter Pufferlösung B gewaschen, um gebundene und freie ³H-AMPA voneinander zu trennen. Nach Messung der Radioaktivität der gebundenen ³H-AMPA im Membranmaterial durch Scintillationszählung wurde durch Auswertung der Verdrängungskurven mittels Regressionsanalyse der K_{I}-Wert bestimmt.

Folgende Ergebnisse wurden erhalten:

| Substanz | Ki [µM] |
|---|---|
| 8-Trifluormethyl-1,2,4-triazolo[1,5-c]chinazolin-5-on-2-carbonsäure | 1,4 |
| 8,10-Dichlor-1,2,4-triazolo[1,5-c]chinazolin-5-on-2-carbonsäure | 1,3 |
| 8-Nitro-1,2,4-triazolo[1,5-c]chinazolin-5-on-2-carbonsäure | 2,6 |

### 2. Bindung von ³H-5,7-Dichlorkynurensäure

Für die Präparation des Membranmaterials wurden frisch entnommene Rattengroßhirne zusammen mit dem ca. 10fachen Volumen einer Pufferlösung A aus 50 mM TRIS-HCl und 10 mM EDTA - pH 7,4 - homogenisiert. Die Suspension wurde 20 Minuten bei 48 000 g zentrifugiert. Nach Abtrennung der überstehenden Flüssigkeit wurde das im Bodensatz enthaltene Membranmaterial zweimal durch Suspendieren in der Pufferlösung A und anschließendes, jeweils 20 minütiges Zentrifugieren und Suspendieren gewaschen. Nach erneutem Suspendieren der Membrane in der Pufferlösung A und Einfrieren in flüssigem Stickstoff wurde die Suspension wieder bei 37°C aufgetaut und nach einem weiteren Waschvorgang 15 Minuten bei 37°C inkubiert. Anschließend wurde das Proteinmaterial viermal durch Zentrifugieren und Suspendieren gewaschen und bis zur Verwendung bei -70°C eingefroren.

Für den Bindungstest wurde das bei 37°C aufgetaute Proteinmaterial zweimal durch Zentrifugieren bei 48 000 g (20 Minuten) und anschließendes Suspendieren in einer Pufferlösung B aus 50 mM TRIS-HCl - pH 7,4 - gewaschen. Anschließend wurden 0,15 mg Membranmaterial, 0,3 µCi ³H-5,7-Dichlorkynurensäure (16 Ci/mmol) sowie Verbindung I in 1 ml Pufferlösung B gelöst und 30 Minuten auf Eis inkubiert. Die inkubierte Lösung wurde 2 Minuten bei 150 000 g zentrifugiert. Nach Abtrennung der überstehenden Flüssigkeit wurden die Bodensätze zweimal mit je 1,5 ml kalter Pufferlösung B suspendiert. Nach Messung der Radioaktivität der an die Membranen gebundenen ³H-5,7-Dichlorkynurensäure im Bodensatz ergab sich der K_{I}-Wert durch Auswertung der Verdrängungskurven mittels Regressionsanalyse.

Folgende Ergebnisse wurden ermittelt:

| Substanz | Ki [µM] |
|---|---|
| 8,9-Dimethyl-1,2,4-triazolo[1,5-c]chinazolin-5-on-2-carbonsäure | 0,09 |
| 8,10-Dichlor-1,2,4-triazolo[1,5-c]chinazolin-5-on-2-carbonsäure | 0,2 |
| 8,9-Dimethyl-1,2,4-triazolo[1,5-c]chinazolin-5-on-2-carbonsäureethylester | 0,16 |

Die Verbindungen I sind als Arzneimittelwirkstoffe für die Human- und Veterinärmedizin geeignet. Die Arzneimittelzubereitungen enthalten neben den üblichen Arzneimittelhilfsstoffen eine therapeutisch wirksame Menge an Verbindung I.

Die Arzneimittelzubereitungen können in verschiedenen Applikationsweisen verabreicht werden, wie peroral, parenteral, subkutan, intraperitonal und topisch. So sind Zubereitungsformen wie Tabletten, Emulsionen, Infusions- und Injektionslösungen, Pasten, Salben, Gele, Cremes, Lotionen, Puder und Sprays möglich.

Für die lokale äußere Anwendung, z.B. in Puder und Salben, können die Wirkstoffe in den üblichen Konzentrationen enthalten sein. In der Regel sind die Wirkstoffe in einer Menge von 0,001 bis 5 Gew.-%, vorzugsweise 0,01 bis 0,5 Gew.-%, enthalten.

Bei der inneren Anwendung werden die Präparationen in Einzeldosen verabreicht. In einer Einzeldosis werden pro kg Körpergewicht 0,1 bis 50 mg, vorzugsweise 0,1 bis 10 mg Wirkstoff gegeben. Die Zubereitungen können täglich in einer oder mehreren Dosierungen je nach Art und Schwere der Erkrankung verabreicht werden. Die Tagesdosis liegt in der Regel bei 0,1 bis 100 mg pro kg Körpergewicht bei oraler Gabe und bei 0,01 bis 10 mg pro kg Körpergewicht bei parenteraler Gabe.

Entsprechend der gewünschten Applikationsart enthalten die erfindungsgemäß verwendeten Arzneimittelzubereitungen neben dem Wirkstoff die üblichen Trägerstoffe und Verdünnungsmittel. Für die lokale äußere Anwendung können pharmazeutisch-technische Hilfsstoffe, wie Ethanol, Isopropanol, oxethyliertes, Ricinusöl, oxethyliertes hydriertes Ricinusöl, Polyacrylsäure, Polyethylenglykol, Polyethylenglykolstearat, ethoxylierte Fettalkohole, Paraffinöl, Vaseline und Wollfett verwendet werden. Für die innere Anwendung eignen sich z.B. Milchzucker, Propylenglykol, Ethanol, Stärke, Talk und Polyvinylpyrrolidon.

Ferner können Antioxidationsmittel wie Tocopherol und butyliertes Hydroxyanisol sowie butyliertes Hydroxytoluol, geschmacksverbessernde Zusatzstoffe, Stabilisierungs-, Emulgier- und Bleichmittel enthalten sein.

Die neben dem Wirkstoff in der Zubereitung enthaltenen Stoffe sowie die bei der Herstellung der pharmazeutischen Zubereitung verwendeten Stoffe müssen toxikologisch unbedenklich und mit dem jeweiligen Wirkstoff verträglich sein.

Die Herstellung der Arzneimittelzubereitungen erfolgt in üblicher Weise.

### Beispiel 1

Auf einer Tablettenpresse werden in üblicher Weise Tabletten folgender Zusammensetzung gepreßt:

| | |
|---|---|
| 40 mg | 8,9-Dimethyl-1,2,4-triazolo[1,5-c]chinazolin-5-on-2-carbonsäure |
| 120 mg | Maisstärke |
| 13,5 mg | Gelatine |
| 45 mg | Milchzucker |
| 2,25 mg | Aerosil® (chemisch reine Kieselsäure in submikroskopisch feiner Verteilung) |
| 6,75 mg | Kartoffelstärke (als 6 %iger Kleister) |

### Beispiel 2

In üblicher Weise werden Dragees folgender Zusammensetzung hergestellt:
- 20 mg: 8,9-Dimethyl-1,2,4-triazolo[1,5-c]chinazolin-5-on-2-carbonsäure
- 60 mg: Kernmasse
- 60 mg: Verzuckerungsmasse

Die Kernmasse besteht aus 9 Teilen Maisstärke, 3 Teilen Milchzucker und 1 Teil Luviskol® VA 64 (Vinylpyrrolidon-Vinylacetat-Mischpolymerisat 60:40, vgl. Pharm. Ind. 1962, 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

### Beispiel 3

10 g 8,9-Dimethyl-1,2,4-triazolo[1,5-c]chinazolin-5-on-2-carbonsäure werden in 5000 ml Wasser unter Zusatz von NaCl gelöst und mit 0,1 N NaOH auf pH 6,0 eingestellt, so daß eine blutisotonische Lösung entsteht. Jeweils 5 ml dieser Lösung werden in Ampullen gefüllt und sterilisiert.

## Patentansprüche

1. Verwendung von Triazolochinazolinen der Formel I, 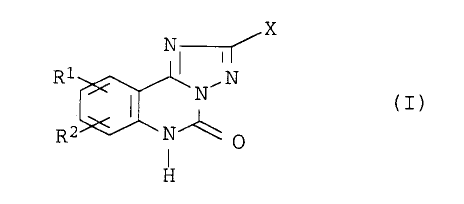 in der
X eine Carboxylgruppe gegebenenfalls in Form ihres Salzes mit einem physiologisch verträglichen Amin- oder Metallkation oder den Rest 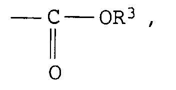 worin R³ einen C₁₋₈-Alkylrest bedeutet,
R¹ ein Wasserstoff-, Chlor- oder Bromatom, oder einen Trifluormethyl-, Nitro-, oder C₁₋₅-Alkylrest und
R² ein Chlor- oder Bromatom, oder einen Trifluormethyl-, Nitro- oder C₁₋₅-Alkylrest
bedeuten sowie deren physiologisch verträglichen Säureadditionssalzen zur Herstellung von Arzneimitteln zur Behandlung zentralnervöser Erkrankungen.

## Claims

1. The use of triazoloqinazolines of the formula I 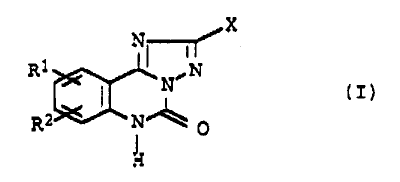 where
X is carboxyl, where appropriate in the form of its salt with a physiologically tolerated amine cation or metal cation, or the radical 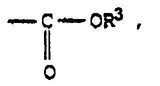 in which R³ is C₁₋₈-alkyl,
R¹ is hydrogen, chlorine or bromine, or trifluoromethyl, nitro or C₁₋₅-alkyl and
R² is chlorine or bromine, or trifluoromethyl, nitro or C₁₋₅-alkyl
and their physiologically tolerated acid addition salts for the production of drugs for the treatment of central nervous disorders.

## Revendications

1. Utilisation de triazoloquinazolines de formule I, 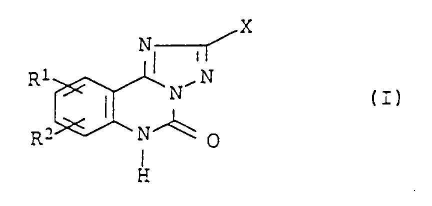 où
X représente un groupe carboxyle éventuellement sous la forme de son sel avec un cation d'amine ou de métal physiologiquement acceptable ou le reste 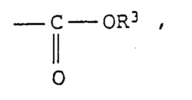 où R³ désigne un reste alkyle en C₁₋₈,
R¹ désigne un atome d'hydrogène, de chlore ou de brome, ou un reste trifluorométhyle, nitro ou alkyle en C₁₋₅ et
R² représente un atome de chlore ou de brome, ou un reste trifluorométhyle, nitro ou alkyle en C₁₋₅,
ainsi que de leurs sels d'addition d'acide physiologiquement acceptables pour la préparation de médicaments pour le traitement des affections du système nerveux central.
